# EUROPEAN PATENT APPLICATION

(11) **EP 2 586 864 A1**
(43) Date of publication of application: **01.05.2013**
(21) Application number: 11186912.9
(22) Date of filing: 27.10.2011
(51) Int. Cl.: C12N 15/113, A61K 31/7088, A61K 31/713

(54) **Upregulating of miR-15b for preventing cellular senescence and tissue aging**

(71) Applicant: IUF Leibnitz-Institut für Umweltmedizinische Forschung gGmbH, 40225 Düsseldorf (DE)
(72) Inventor: Krutmann, Jean, 40237 Düsseldorf (DE); Piekorz, Roland, 40959 Düsseldorf (DE)
(74) Representative: von Kreisler Selting Werner

(57) **Abstract**

The present invention provides compounds for upregulation of miR-15b, which in turn decreases cellular SIRT4 expression, and which are thus suitable for preventing or treating cellular senescence and tissue aging.

## Description

The present invention provides compounds for upregulation of miR-15b, which in turn decreases cellular SIRT4 expression, and which are thus suitable for preventing or treating cellular senescence and tissue aging.

### Background of the Invention

Human premature skin aging (=photoaging) is driven by UV light as major environmental noxae (Krutmann and Gilchrest, Photoaging of skin. In: Skin Aging (Gilchrest BA, Krutmann J, eds), (2006)). Whereas UVA radiation penetrates the human skin more deeply and effects both the epidermis and dermis, UVB acts preferentially on the epidermis as proliferative compartment where it elicits among the production and release of soluble factors like proteolytic enzymes also DNA damage (Fisher et al., N Engl J Med 337:1419-28, (1997); Krutmann and Schroeder, J Investig Dermatol Symp Proc 14:44-9, (2009)). As a consequence, DNA damage triggered by UVB light or other extrinsic factors, including oxidative stress or cytotoxic drugs, causes cellular aging referred to as stress-induced premature senescence (SIPS) (Campisi and d'Adda di Fagagna, Nat Rev Mol Cell Biol 8:729-40, (2007)). This phenotype is typically associated with and dependent on mitochondrial dysfunction (Ben-Porath and Weinberg, Int J Biochem Cell Biol 37:961-76, (2005); Collado and Serrano, Nat Rev Cancer 6:472-6, (2006); Moiseeva et al., Mol Cell Biol 29:4495-507, (2009); Passos et al., Mol Syst Biol 6:347, (2010)). In addition, human cells display an intrinsic type of senescence (replicative senescence) which is based on the progressive erosion of telomeres. Senescent cells are characterized by several features which distinguish them from quiescent cells reversibly arrested in the G₁ phase of the cell cycle (Campisi and d'Adda di Fagagna, Nat Rev Mol Cell Biol 8:729-40, (2007); Chuaire-Noack, Int J Morphol 28:37-50, (2010)). In particular, upon senescence cells adopt morphological changes like a flattened morphology and increased granularity, formation of senescence associated heterochromatic foci (SAHF) linked to the repression of proliferative genes and hence DNA synthesis, and increased SA-β-galactosidase (SA-βgal) expression based on an expansion of the lysosomal compartment. It was recently shown that premature senescence can also be induced as a consequence of mitotic spindle stress elicited by cellular depletion of the centrosomal protein TACC3 (Schmidt et al., Oncogene 29:6184-92, (2010)). In this model system, centrosomal and spindle dysfunction were found to modulate post-mitotic senescence through a p53-p21^{WAF} dependent pathway (Schmidt et al., Cell Cycle 9:4469-73., (2010)).

Sirtuins comprise a conserved protein family of NAD⁺-dependent deacetylases or, in the case of SIRT4, ADP-ribosyltransferases (Haigis and Sinclair, Annu Rev Pathol 5:253-95, (2010); Michishita et al., Mol Biol Cell 16:4623-35, (2005)). The prototype sirtuin, SIRT1, plays diverse and senescence/aging-associated, but currently not fully understood roles in metabolism and stress biology. Downregulation or inhibition of SIRT1 is associated with metabolic dysregulation and antagonization of anti-aging effects linked to calorie restriction, impaired DNA repair, and apoptosis in the context of cellular stress responses (Donmez and Guarente, Aging Cell 9:285-90, (2010); Guarente, Cold Spring Harb Symp Quant Biol 72:483-8, (2007)). Moreover, SIRT1 contributes to telomere maintenance (Palacios et al., J Cell Biol 191:1299-313, (2010)) and is downregulated during cellular senescence (Sasaki et al., Aging Cell 5:413-22, (2006)). In contrast to all other sirtuins localized in the cytosol or nucleus, SIRT3, SIRT4 and SIRT5 are exclusively found in the mitochondrium, an organelle linking energy metabolism and aging (Verdin et al., Trends Biochem Sci 35:669-75, (2010)). Metabolic enzymes as specific substrates of mitochondrial SIRT proteins (mtSIRTs) have been identified (Haigis and Sinclair, Annu Rev Pathol 5:253-95, (2010)), but the putative roles of mtSIRTs in intrinsically and extrinsically induced cell and tissue aging are currently poorly understood. In this context, little is known about the mechanisms involved in regulation of expression of mtSIRTs during cellular and organismal aging.

The exact molecular mechanisms, by which UV radiation leads to skin aging, remain to be clarified. Mitochondrial dysfunction and production of reactive oxygen species (ROS) are implicated in cellular senescence (Passos et al., Mol Syst Biol 6:347, (2010)) and photoaging of the skin ("defective powerhouse model") (Krutmann and Schroeder, J Investig Dermatol Symp Proc 14:44-9, (2009)). Moreover, a decreased mitochondrial membrane potential accompanied by increased ROS levels has been recently observed in skin fibroblasts from aged donors (Koziel et al., J Invest Dermatol 131:594-603, (2011)). Given these findings we analyzed in this study the expression and regulation of mtSIRTs in various models of cellular and tissue aging. Interestingly, in all models investigated, including the photo-aged skin, SIRT4 was uniquely upregulated, and this upregulation was always accompanied by a decreased expression of the miRNA miR-15b. Accordingly, compensation of endogenous miR-15b function by transfected miR-15b "mimics" was sufficient to inhibit SIRT4 upregulation during cellular senescence. Thus, miR-15b functions as a negative regulator of SIRT4 expression to antagonize mitochondrial dysfunction and hence premature senescence and tissue aging.

### Summary of the Invention

When searching for a possible link between mitochondrial sirtuins (mtSIRTs) and premature senescence and skin aging, SIRT4 was identified, the overexpression of which negatively impacts on mitochondrial oxidative capacity, as uniquely and consistently upregulated in various human cellular and tissue aging models. In particular, SIRT4 was upregulated in extrinsically (= photo) aged vs. intrinsically aged human skin as well as in CD34⁺ hematopoietic progenitors isolated from adults as compared to umbilical cord blood. Moreover, SIRT4 mRNA levels were significantly increased in human dermal fibroblasts which had been repetitively exposed to ultraviolet radiation or in premature senescence triggered by mitotic spindle stress or by ionizing radiation. Interestingly, in all cellular and tissue aging models analyzed, including the photo-aged human skin, upregulation of SIRT4 expression was always associated with decreased levels of miR-15b, a microRNA with a highly conserved binding site within the 3'-untranslated region of the *SIRT4* gene in vertebrates. Moreover, transfection of oligonucleotides mimicking endogenous miR-15b function was sufficient to prevent SIRT4 upregulation in premature senescence. Thus, miR-15b acts as a negative regulator of SIRT4 expression to antagonize mitochondrial dysfunction and hence cellular senescence as well as tissue aging, in particular photoaging of the skin.

The invention thus provides
(1) a compound for upregulating cellular miR-15b expression, a pharmaceutical composition and medicament comprising said compound for use in preventing or treating cellular senescence and tissue aging;
(2) a preferred embodiment of (1) above, wherein the compound is a miR-15b miRNA mimic;
(3) the use of the compound of (1) or (2) above for preparing a medicament for preventing or treating cellular senescence and tissue aging;
(4) An *in-vitro* method for preventing or treating cellular senescence and tissue aging, which comprises contacting the cells or tissue with the compound for upregulating cellular miR-15b expression as defined in (1) or (2) above;
(5) a pharmaceutical or cosmetical method for preventing or treating cellular senescence and tissue aging in a subject which comprises administering to the subject an effective amount of the compound for upregulating cellular miR-15b expression as defined in (1) or (2) above; and
(6) a cosmetical composition for preventing or treating cellular senescence and tissue aging comprising the compound for upregulating cellular miR-15b expression as defined in (1) or (2) above.

The above preventing or treating of cellular senescence and tissue aging refers to all kinds of cells and tissues of vertebrate/mammalian origin and preferably refers to preventing or treating of skin aging, notably in human beings.

### Description of the Figures

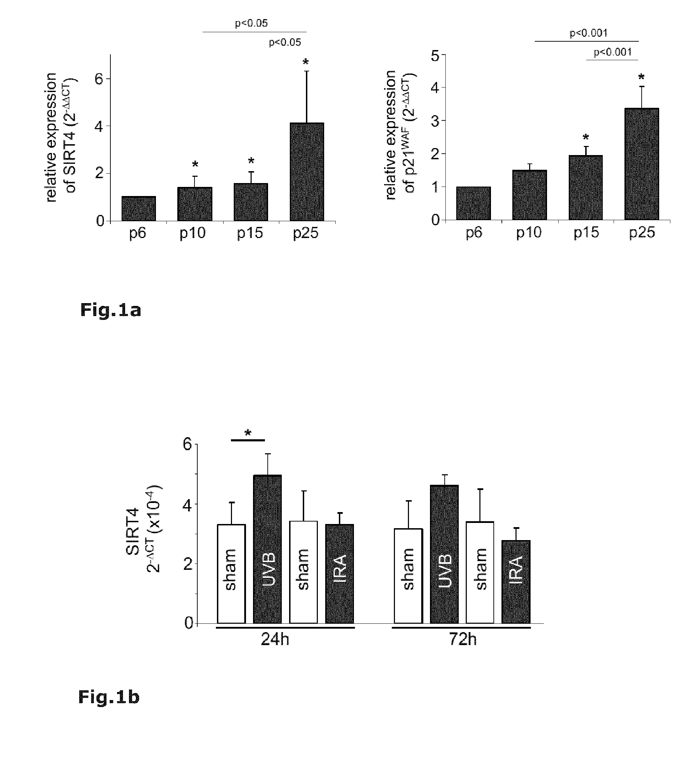

Figure 1: Increased expression of the mitochondrial sirtuin SIRT4 in human fibroblasts undergoing replicative or UVB-induced senescence. (a) Concomitant upregulation of SIRT4 and p21^{WAF} expression during replicative senenescence. Human foreskin fibroblast lines (from n=4 donors; mean ± s.d.; *p<0.05) were analysed at increasing passage numbers for SIRT4 and p21^{WAF} mRNA levels by quantitative real-time polymerase chain reaction (qPCR; suppl. Material & Methods). (b) Primary human dermal fibroblasts (n=4 donors; mean ± s.d.; *p<0.05) were subjected to ultraviolet irradiation (100 J/m² UVB per day) for a total of five days. Cells were harvested 24 and 72 h following the last irradiation and SIRT4 mRNA levels were determined by qPCR.

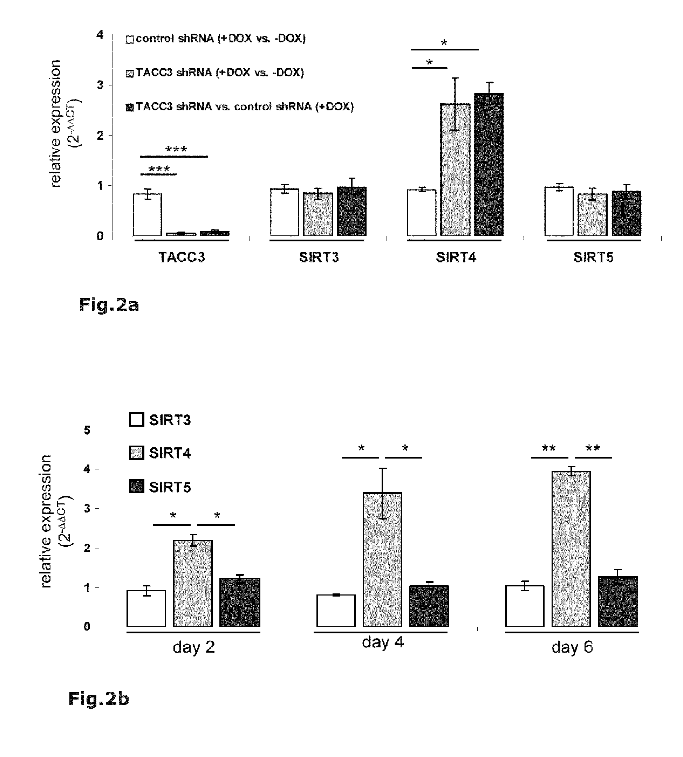

Figure 2: Selective upregulation of SIRT4 expression among the mitochondrial SIRT isoforms in cellular models of stress-induced premature senescence. (a) Increased SIRT4 mRNA levels in MCF7 cells driven into premature senescence through shRNA-mediated depletion of the centrosomal protein TACC3 (Schmidt S., et al., Oncogene 29:6184-92 (2010)) (analysis on day 4 upon DOX treatment which induces control or TACC3 shRNA expression; mean ± s.d. from three independent experiments; *p<0.05; **p<0.01). (b) Upregulation of SIRT4 mRNA levels in MCF7 cells 2, 4 and 6 days following ionizing radiation (single dose of 20 Gy; mean±s.d. from three independent experiments; *p<0.05; **p<0.01). mRNA levels were determined by quantitative real-time polymerase chain reaction.

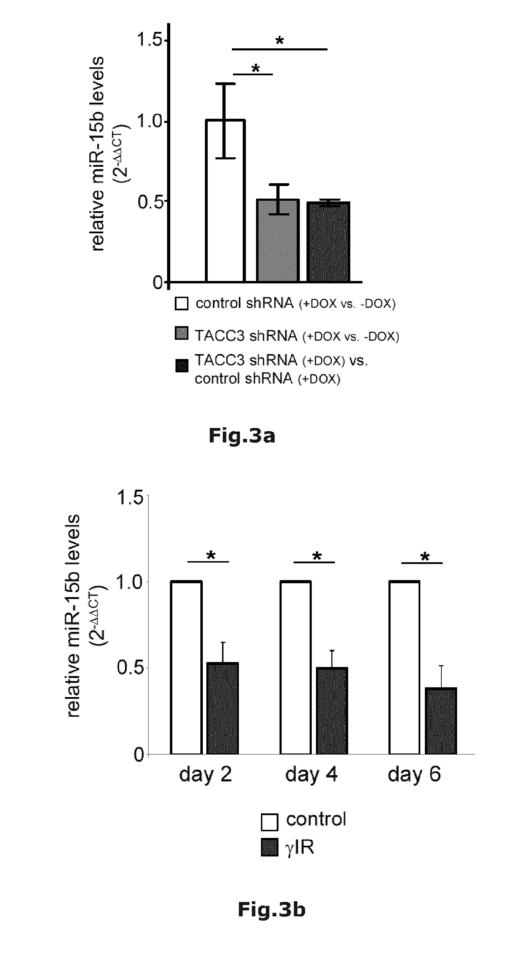

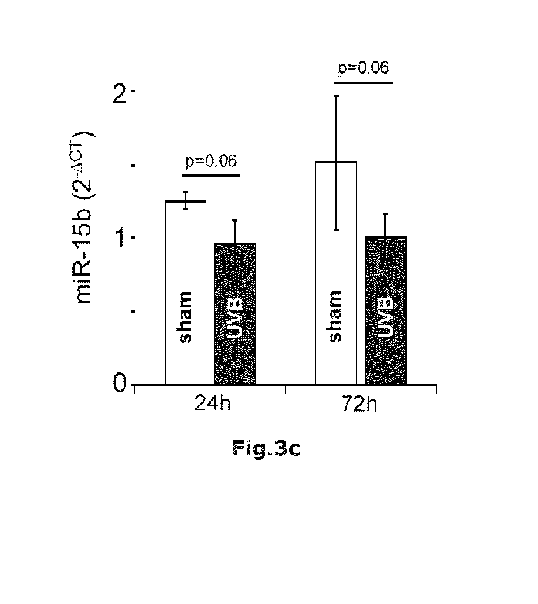

Figure 3: Cellular levels of miR-15b are decreased concomitant with upregulated SIRT4 gene expression in various aging models. (a) Decreased miR-15b mRNA levels in MCF7 cells following induction of senescence through TACC3 depletion (analysis on day 4 of DOX-induced TACC3 shRNA expression; mean ± s.d. from three independent experiments; *p<0.05). (b) Analysis of miR-15b levels in MCF7 cells following ionizing radiation (application of 20 Gy at day 0; mean ± s.d. from three independent experiments). (c) Primary human dermal fibroblasts (n=4 donors) were subjected to ultraviolet irradiation (100 J/m² UVB per day) for a total of five days. Cells were harvested 24 and 72 h following the last irradiation and miR-15b levels were determined by qPCR (mean ± s.d).

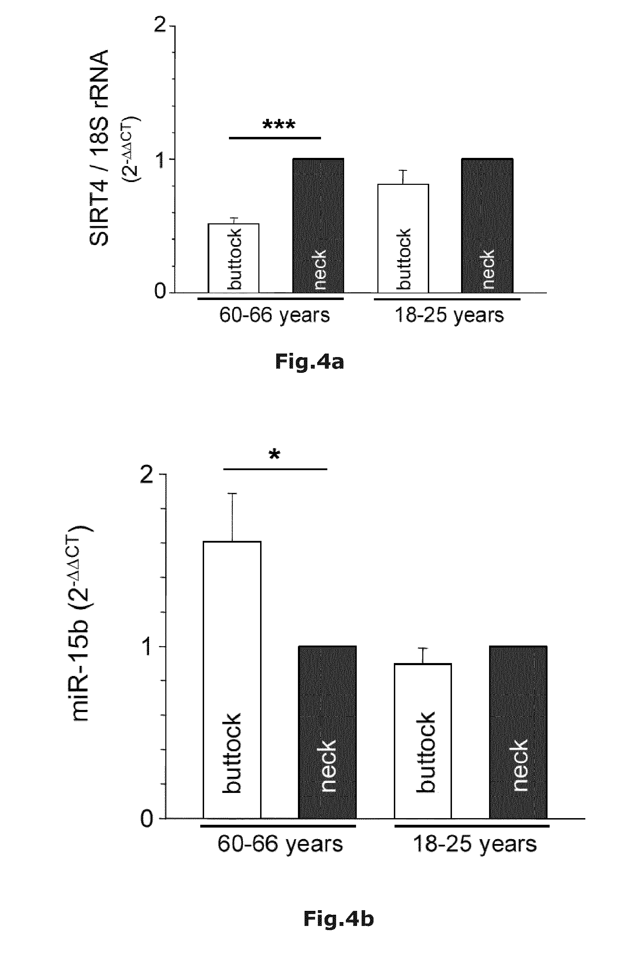

Figure 4: Increased SIRT4 expression is inversely associated with decreased miR-15b levels in human photo-aged skin. (a) SIRT4 expression analysis in human skin samples obtained from neck vs. buttock skin of the same individual (mean ± s.e.m; ***p<0.001). Probands from two age groups (18-25 years and 60-66 years; n=15-16 per group) were compared. (b) miR-15b expression analysis in human skin samples obtained from neck vs. buttock skin of the same individual (mean ± s.e.m; *p<0.05). Probands from two age groups (18-25 years and 60-66 years; n=9-10 per group) were compared.

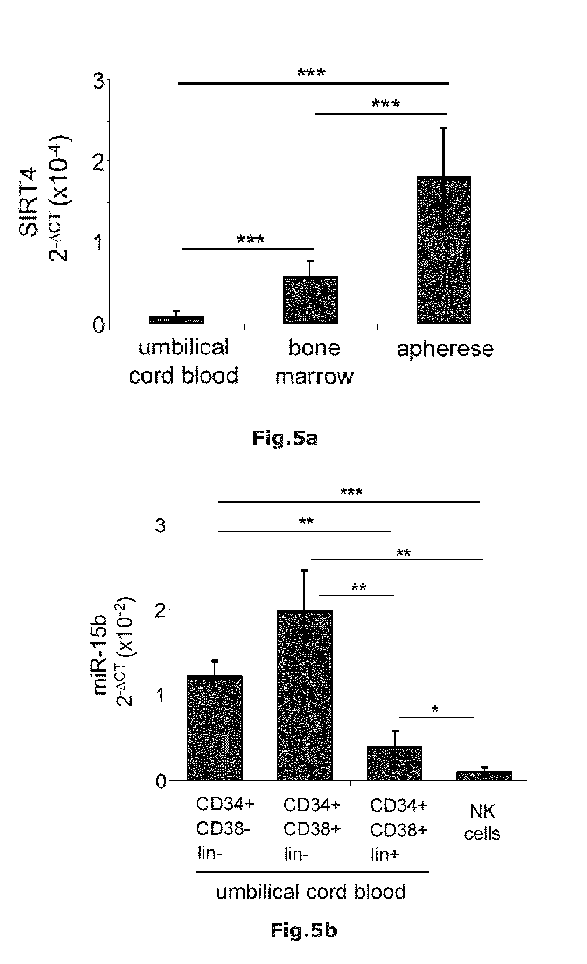

Figure 5: Hematopoietic progenitor cells display an age-associated increase in SIRT4 expression accompanied with descreased miR-15b levels. (a) Upregulation of SIRT4 mRNA levels in aged CD34⁺/lineage marker negative (lin⁻) hematopoietic progenitor cells as compared to cells isolated from umbilical cord blood (cord blood: n=9; bone marrow: n=5, age range 27-47 years; progenitor cells isolated by apheresis from G-CSF mobilized blood: n=17, age range 25-55 years; mean ± s.d.; ***p<0.001). (b) Analysis of miR-15b levels in CD34⁺/lineage marker negative (lin⁻) hematopoietic progenitor cells from cord blood as compared to determined lin⁺ cells and natural killer (NK) cells (n=3, mean ± s.d.; *p<0.05; **p<0.01; ***p<0.001).

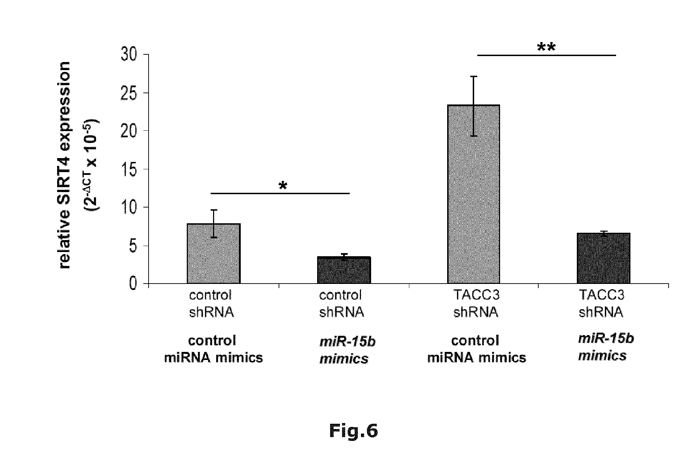

Figure 6: Suppression of senescence-associated SIRT4 upregulation by oligonucleotides mimicking the function of endogenous miR-15b. Extrinsically applied miR-15b mimics inhibit basal SIRT4 mRNA levels and prevent their upregulation in MCF7 cells driven into premature senescence through shRNA-mediated TACC3 depletion (analysis on day 4 upon DOX treatment which induces control shRNA or TACC3 shRNA expression; n=3 independent experiments, mean ± s.d.; *p<0.05; **p<0.01).

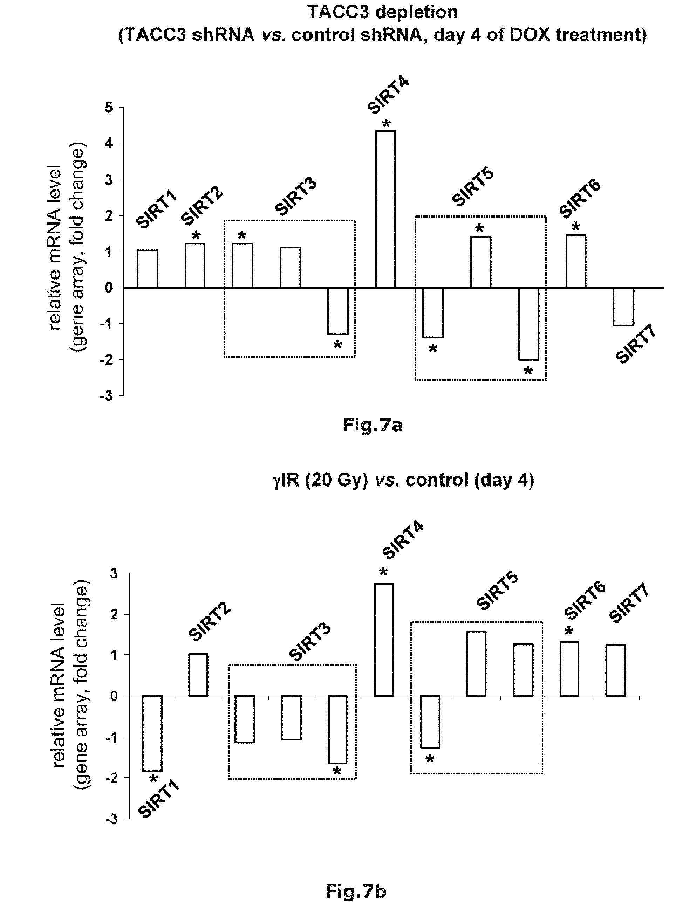

Figure 7: Microarray based expression analysis of SIRT isoforms in MCF7 cells at day 4 of DOX (doxycyclin)-induced TACC3 shRNA expression (a) or four days upon γ-irradiation using 20 Gy as single dosis (b). For SIRT3 and SIRT5, three oligos each were detected on the original arrays with rather inconsistent expression changes. Only SIRT4 mRNA levels were significantly and consistently upregulated both in TACC3-depleted and γ-irradiated cells (mean ± s.d. from four replicates of TACC3-depleted or γ-irradiated cells vs. controls; *p<0.05).

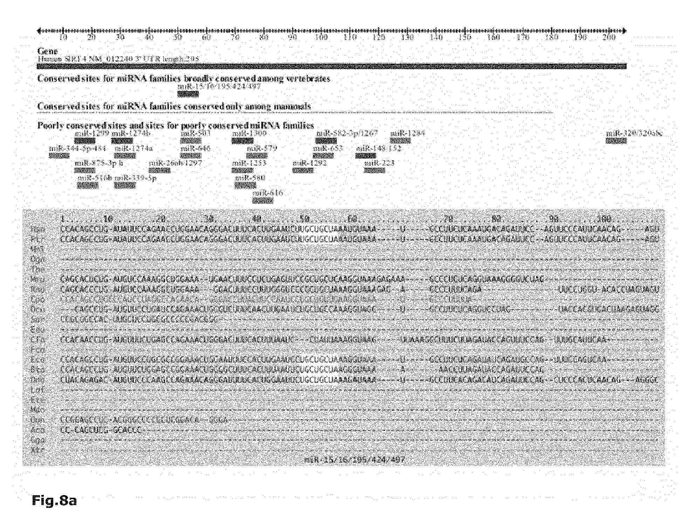

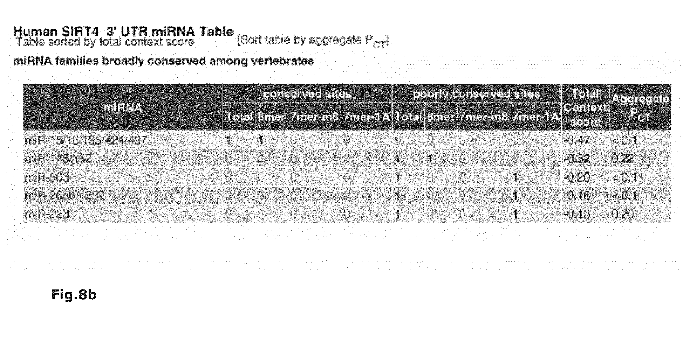

Figure 8: Prediction of miRNA binding sites using the TargetScan software. (a) Overview of candidate miRNAs binding to the 3'-untranslated region (3'-UTR) of the *SIRT4* gene in vertebrates. The putative binding site for miR-15/16/195/424/497 (all miRNAs display the same seed region of 8 bp) is highly conserved among vertebrates (Hsa, Homo sapiens). Among these miRNAs, only miR-15b was downregulated in TACC3-depleted MCF7 cells undergoing premature senescence (Fig. 2). (b) Comparison between conserved and poorly conserved miRNA sites in the human *SIRT4* gene. Algorithms used by TargetScan (www.targetscan.org) have been described by Friedman R.C., et al., Genome Res. 19:92-105 (2009), Grimson A., et al., Mol Cell. 27:91-105 (2007) and Lewis B.P., et al., Cell. 120:15-20 (2005).

### Detailed Description of the invention

The compound for upregulating cellular miR-15b expression and composition comprising said compound for use in preventing or treating cellular senescence and tissue aging according to aspect (1) of the invention may be any compound that upregulates miR-15b expression.

It is preferred that such compound that upregulates miR-15b expression is a miR-15b a miRNA mimic. Such miR-15b miRNA mimic binds to the miR-15b binding site UGCUGCUA or the complement thereof within the 3'-UTR of the *SIRT4* gene. Particular miR-15b miRNA mimic comprises a nucleotide sequence having the sequence of 5'-UAGCAGCACAUCAUGGUUUACA (SEQ ID NO:14), 5'-CGAAUCAUUAUUUGCUGCUCUA (SEQ ID NO:15), or a complement thereof, most preferably has the sequence of SEQ ID NOs 14 or 15 or a complement thereof. Such miR-15b miRNA mimic may be comprised in a nucleic acid construct, vector or viral vector or may be encoded by such construct or vector.

The "compound for upregulating cellular miR-15b expression" within the meaning of the invention refers to an agent or medicament containing the compound as defined hereinbefore. Similarly, the "pharmaceutical and cosmetical compositions comprising the compound" within the meaning of the invention refers to pharmaceutical and cosmetical compositions which may comprise further active agents. All of said medicaments, reagents and compositions may further comprise pharmacologically or cosmetically acceptable carrier, excipients and the like.

The compounds, medicaments, reagents and compositions are for the in-vivo use in preventing or treating cellular senescence and tissue aging in vertebrates such as mammals (including human beings and animals) and are also suitable for the in vitro treatment of cells and tissue culture derived from such vertebrate and for cosmetic treatment of skin. This is reflected in aspects (3) to (6) of the invention.

Notably, aspect (3) of the invention relates to the use of the compound for downregulating cellular miR-15b expression as defined hereinbefore for preparing a medicament for preventing or treating cellular senescence and tissue aging.

Further, aspect (4) of the invention relates to an in-vitro method for preventing or treating cellular senescence and tissue aging in-vivo and in-vitro which comprises contacting the cells or tissue with the compound for downregulating cellular miR-15b expression as defined hereinbefore.

Further, aspect (5) of the invention relates to a method for preventing or treating cellular senescence and tissue aging in a subject which comprises administering to the subject an effective amount of the compound for downregulating cellular miR-15b expression as defined hereinbefore. The subject being the vertebrate as defined hereinbefore.

Finally, aspect (6) of the invention relates to a cosmetical composition for preventing or treating cellular senescence and tissue aging comprising the compound for upregulating cellular miR-15b expression as defined in any one of claims 1 to 5.

In the present study, the role of mtSIRTs in aging by analyzing their expression during cellular senescence and tissue aging was determined. Initially, it was observed that SIRT4 mRNA levels were upregulated at later passages during replicative senescence of human foreskin fibroblasts, comparable to the parallelly increased expression of the cell cycle inhibitor and senescence effector p21^{WAF} (Figure 1a). The observed increase in SIRT4 mRNA levels was not specific to intrinsic aging, because SIRT4 transcripts were also found at elevated levels in primary human dermal fibroblasts, in which senescence has been induced extrinsically by repetitive exposure to low, sublethal doses of ultraviolet radiation (Chainiaux et al., Int J Biochem Cell Biol 34:1331-9, (2002); Debacq-Chainiaux et al., J Cell Sci 118:743-58, (2005)) (Figure 1b).

To test whether the senescence-associated SIRT4 upregulation was cell-type specific or rather of general relevance, we next employed additional cellular models of premature senescence. This analysis was combined with a genearray-based expression screen of all SIRT isoforms followed up by reassessment of the expression of mtSIRTs using qPCR. Interestingly, among the seven sirtuin family members, and in particular among the mtSIRTs SIRT3, SIRT4 and SIRT5, only SIRT4 was consistently and significantly upregulated in MCF7 cells undergoing premature senescence. This was observed regardless of whether senescence was triggered by mitotic spindle stress through depletion of the centrosomal protein TACC3 (Schmidt et al., Cell Cycle 9:4469-73, (2010)) or by treatment with ionizing radiation (γIR) (Essmann et al., Cancer Res 64:7065-72, (2004)) (suppl. Figure 1 and Figure 2a, b). Thus, these findings indicate that cellular senescence, either of replicative or premature nature, is accompanied in a cell type-independent manner by an increased SIRT4 expression.

From a functional point of view it appears that SIRT3 and SIRT5 promote, whereas SIRT4 antagonizes mitochondrial energy production and homeostasis (Verdin et al., Trends Biochem Sci 35:669-75, (2010)), suggesting that SIRT3 and SIRT5 antagonize metabolic abnormalities relevant in cellular and organismal aging, whereas SIRT4 may act in an opposite way. In particular, SIRT3 deacetylates and therefore activates mitochondrial HMG-CoA synthetase 2 required for ketone body production (Shimazu et al., Cell Metab 12:654-61, (2010)) and functions as a metabolic tumor suppressor (Kim et al., Cancer Cell 17:41-52, (2010)). Moreover, a recent report demonstrates that SIRT3 deficiency causes a severe dysregulation of mitochondrial protein acetylation which then contributes to the development of the metabolic syndrome (Hirschey et al., Mol Cell doi:10.1016/j.molcel.2011.07.019, (2011)). SIRT5 has been described to positively regulate the urea cycle (Nakagawa et al., Cell 137:560-70, (2009)). In contrast, SIRT4 negatively impacts on gene expression and various metabolic processes in mitochondria. Accordingly, knock down of SIRT4 *in vivo* leads to an improved fatty acid oxidation associated with an increased SIRT1 expression and elevated pAMPK levels (Nasrin et al., J Biol Chem 285:31995-2002, (2010)). Moreover, SIRT4 impedes mitochondrial oxidative capacity and function through the inhibition of its target glutamate dehydrogenase (GDH) normally linking amino acid metabolism with oxidative phosphorylation (Haigis et al., Cell 126:941-54, (2006)). Interestingly, GDH activity decreases during aging in lymphocytes (Kravos and Malesic, Neurodegener Dis 7:239-42, (2010)), though is it currently unclear to which extent inhibition of GDH activity is primarily mediated by increased SIRT4 levels and whether this scenario also occurs in other tissues during aging. In aggregate, these studies together with the present finding that increased SIRT4 levels are associated with aging indicate that extrinsic factors induce cell and tissue aging by upregulating SIRT4 expression and subsequent inhibition of mitochondrial function.
Little is currently known about the regulation of SIRT4 expression. A major feature of senescent cells is their altered gene expression profile, which leads to growth arrest, apoptosis resistance, and induction of a senescence associated secretory program (SASP) (Collado and Serrano, Nat Rev Cancer 6:472-6,

(2006); Coppe et al., Annu Rev Pathol 5:99-118, (2010)). In this regard, a role of small non-coding silencing RNAs (miRNAs) has been proposed in various senescence and aging model systems (Bhaumik et al., Aging (Albany NY) 1:402-11, (2009); Hackl et al., Aging Cell 9:291-6, (2010); Hong et al., Cancer Res 70:8547-57, (2010); Lafferty-Whyte et al., Biochim Biophys Acta 1792:341-52,

(2009)). miRNAs are potent regulators of gene expression networks by either degrading or translationally repressing one or more target mRNAs. This enables cells to coordinate and fine-tune a variety of cellular processes or to modulate antagonistic phenotypes like malignant proliferation *vs.* senescence/cellular aging. A prime example is the increased *vs.* decreased levels of cell cycle regulating miRNAs from the miR-17-92 cluster, which are associated with malignant proliferation or oncogene-induced senescence, respectively (Grillari et al., Biogerontology 11:501-6., (2010); Hong et al., Cancer Res 70:8547-57, (2010)).

In order to assess the potential role of miRNAs in senescence-associated SIRT4 upregulation, we next performed a global, microarray-based comparison of corresponding miRNA- and mRNA expression profiles followed by relative miRNA quantification using qPCR in our model systems. Among the miRNAs analyzed, we observed a robust and significant downregulation of miR-15b in TACC3 depleted or γ-irradiated senescent MCF7 cells (Figure 3a, b), concomitant with the parallel upregulation of SIRT4 expression (Figure 2). Similarly, and again in accordance with the SIRT4 expression status (Figure 1b), miR-15b levels were also decreased in extrinsically aged primary human dermal fibroblasts (Figure 3c). Lastly, consistent with our findings, Hackl *et al.* observed upto twofold decreased miR-15b levels in various models of organismal as well as replicative aging (Hackl et al., Aging Cell 9:291-6, (2010)), the latter being in accordance with the increased SIRT4 expression in replicatively aged human fibroblasts (Figure 1a).

Having established a novel and inverse correlation between miR-15b and SIRT4 expression in various aging models, we next addressed whether an analogous scenario exists particularly in human premature skin aging (photoaging). Interestingly, a highly significant increase of SIRT4 transcripts was also measured in human skin biopsies obtained from extrinsically, i.e. photoaged human skin (neck) as compared with intrinsically aged skin (buttock) of the same individuals (age range: 60-66 years) or to skin of young individuals (age range 18-25 years) (Figure 4a). At the same time, miR-15b levels were significantly lower in the photoaged skin samples of older individuals as compared with their intrinsically aged skin counterpart (buttock) (Figure 4b). In contrast, in preliminary studies we did not detect any significant expression changes for SIRT3 or SIRT5 in photoaged skin from old individuals (neck *vs.* buttock; data not shown). This observation is consistant with the unchanged expression of SIRT3 and SIRT5 in the MCF7 senescence model (Figure 2). Thus, a currently unknown and specific role of the miR-15b - SIRT4 axis can be hypothesized not only in premature senescence at the cellular level, but also in photoaging of human skin, and thus in organ aging. Interestingly, in this regard, increased SIRT4 levels were not restricted to skin aging, but were also observed in aging of human hematopoietic progenitor cells. We detected significantly higher SIRT4 transcript levels in CD34⁺ hematopoietic progenitors isolated from adults as compared to cells isolated from newborn umbilical cord blood (Figure 5a). At the same time, levels for miR-15b were significantly higher in CD34⁺/lin⁻ progenitor cells isolated from umbilical cord blood as compared to e.g. natural killer (NK) cells from peripheral blood of adult probands (Figure 5b). miR-15b was the only regulated and senescence-linked miRNA in our miRNA/mRNA screen with a uniquely conserved binding site within the 3'-untranslated region (UTR) of the *SIRT4* gene in vertebrates (suppl. Figure 2). It should be noted that this binding site can be also targeted by additional miRNAs recognizing the same seed sequence (miR16/195/424/497; suppl. Figure 2) (Finnerty et al., J Mol Biol 402:491-509, (2010)). However, we did not detect altered levels of these miRNAs in our microarray screen (data not shown). Moreover, miR-195 levels are elevated rather than downregulated upon replicative and premature senescence (Maes et al., J Cell Physiol 221:109-19, (2009)). Overall, miR-15b is subject to global cell stress and p53 regulation (Finnerty et al., J Mol Biol 402:491-509, (2010)), and contrary to cellular senescence increased miR-15b expression correlates with tumor cell proliferation and apoptosis (Satzger et al., Int J Cancer 126:2553-62, (2010)). Importantly, recent functional studies identified miR-15b as a negative modulator of cellular ATP levels and mitochondrial function via its target ADP-ribosylation factor-like 2 (ArI2) (Nishi et al., J Biol Chem 285:4920-30, (2010)). SIRT4 and Arl2 are not the only targets transcriptionally regulated during cellular senescence and implicated in the regulation of mitochondrial energy metabolism. Using the aging mouse brain model, Li et al. observed an upregulation of a panel of miRNAs predicted to target key genes involved in oxidative phosphorylation, including the mitochondrial complex IV and F0F1 ATPase (Li et al., Neurobiol Aging 32:944-55, (2009)).

Lastly, to functionally link miR-15b to the regulation of SIRT4 mRNA levels, we next tested the possibility to influence cellular SIRT4 mRNA levels by exogenously applied oligonucleotides mimicking endogenous miR-15b function (miR-15b "mimics"). Employing transfection studies using the MCF7 model of spindle stress-triggered senescence (Figure 2), we demonstrate that miR-15b mimics downregulate basal SIRT4 mRNA levels and completely prevent senescence-associated upregulation of SIRT4 transcripts (Figure 6). Thus, miR-15b mimics are *per se* sufficient and highly effective in targeting SIRT4 expression, suggesting that additional miRNAs potentially recognizing the 3'-UTR of the *SIRT4* gene are of limited functional relevance for SIRT4 expression.

In conclusion, our results indicate that miR-15b may act as a negative regulator of SIRT4 expression in order to antagonize mitochondrial dysfunction and hence intrinsically and extrinsically induced cell and tissue aging. However, despite the genetic and functional classification of positive or negative roles of mitochondrial sirtuins (Verdin et al., Trends Biochem Sci 35:669-75, (2010)), overexpression of any of the SIRT proteins *per se* does not influence replicative life span of human cells (Michishita et al., Mol Biol Cell 16:4623-35, (2005)). Therefore, SIRT4 may rather take over a modulating role and thereby promote cellular and premature senescence at a later time point, rather than being essential for triggering this process. In particular, deregulation of mitochondrial sirtuins may contribute to the generation of mitochondrial ROS required for maintenance of the senescent phenotype (Passos et al., Mol Syst Biol 6:347, (2010)). Similarly, although recent findings from various animal models suggest that ROS may not be the initial cause of aging (Hekimi et al., Trends Cell Biol 21:569-76, (2011)), dysfunction of mtSIRTs still fits in a model, in which increased mitochondrial ROS may be tightly associated with stress responses linked to age-dependent damage (Hekimi et al., Trends Cell Biol 21:569-76, (2011)). In this line, Koziel *et al.* found a decreased mitochondrial membrane potential accompanied by increased ROS levels in fibroblasts from aged human donors (Koziel et al., J Invest Dermatol 131:594-603, (2011)), further supporting the concept that photoaging of human skin is closely linked to mitochondrial dysfunction of dermal fibroblasts, as previously proposed (Krutmann and Schroeder, J Investig Dermatol Symp Proc 14:44-9, (2009)).

Irrespective, in regard to the role of mtSIRTs in extrinsic aging, future studies are necessary to characterize the mechanistic role of the miR-15b - SIRT4 axis in mitochondrial (patho)physiology and photoaging by employing mouse models deficient in SIRT4 expression and/or function. Furthermore, it remains to be tested whether mitochondrial DNA (mtDNA) mutagenesis is associated with an altered miR-15b - SIRT4 axis.

The invention is further described in the following experiments, which are not to be construed as limiting the invention.

### Examples

### Material and Methods

Cellular TACC3 depletion in MCF7 cells. The centrosomal protein TACC3 was depleted essentially as described (Schmidt et al., Oncogene. 29:6184-6192, (2010); Schneider et al., J Biol Chem. 282:29273-29283, (2007)). Cells were plated one day prior to doxycycline treatment at a density of 3x10³/cm². Expression of control or TACC3 shRNA was induced by adding doxycycline (5 µg/ml) to the culture medium four 4 days.

Ionizing γ-Irradiation (γIR) of MCF7 cells. Trypsinized MCF7 breast carcinoma cells were exposed to γIR (20 Gy) using a Gammacell 1000 Elite irradiator (Nordion International, Inc., Fleurus, Belgium). After irradiation, cells were plated at a density of 3x10³/cm² (control) and 6x10³/cm² (20 Gy) and cultured for 2, 4 and 6 days.

Repetitive UV treatment of human skin fibroblasts. Human dermal fibroblasts were isolated from the foreskin of four different donors (age: 1 to 3 years). Approval had been obtained from the Ethics Committee of the Heinrich-Heine-University. The study has been conducted according to the ethical rules stated in the Declaration of Helsinki Principles and the ICH GCP guideline was followed as applicable. Cells were cultivated in Eagle's minimum essential medium with Earle's salts (MEM, PAA Laboratories, Pasching, Austria) and supplemented with 10% fetal bovine serum (Invitrogen, Karlsruhe, Germany), 1% antibiotics/antimycotics (penicillin, streptomycin, amphotericin B), and 1% glutamine (Invitrogen) and then cultivated on 100 mm plastic culture dishes (Greiner, Solingen, Germany) at 37°C in humidified air with 5% CO₂. Cells were used between passages five and ten and grown to confluency before treatment. Twenty four hours before UV irradiation media were changed to serum-free MEM. Cells were exposed once daily to a single dose of 100 mW/m² ultraviolet radiation from TL20W/12RS SLV (Phillips, Hamburg, Germany) fluorescent bulbs, which primarily emit in the range of 290-315 nm (= UVB) for a period of five days. For irradiation, medium was replaced by phosphate-buffered saline (37°C, Invitrogen). Control cells (sham) were held on RT under similar conditions, but without irradiation. Following the last treatment, cells were cultivated for 24 and 72 hours with serum-free MEM medium at 37°C.

RNA extraction from cultured and irradiated cells and quantitative real-time PCR analysis (mRNA and miRNA). Cell lysates were homogenized using Qiashredder spin columns (Qiagen, Hilden, Germany). Total RNA without small RNAs (> 200 bp) was extracted using the RNeasy Plus Mini Kit (Qiagen, Hilden, Germany) following the standard manufacturers protocol. Total RNA containing small RNAs was isolated using the RNeasy Plus Mini Kit (Qiagen, Hilden, Germany) following the supplementary protocol "Purification of total RNA containing miRNA from animal cells using the RNeasy Plus Mini Kit". Nucleic acid concentrations were measured at 260 nm by spectrophotometry using a NanoDrop 1000 Spectrophotometer (Thermo Scientific, Wilmington, DE, USA). Extracted RNA samples were stored at -80 °C.

For relative quantification of mRNAs, total RNA was reverse transcribed to cDNA using the ImProm-II™ Reverse Transcription System (Promega, Madison, WI, USA) according to the manufacturer's instructions. In brief, up to 1 µg RNA was reverse transcribed to cDNA in a final volume of 20 µl using Oligo (dT)₁₅ primers (0.5 µg/reaction) and 3.75 mM MgCl₂. Each quantitative real-time PCR reaction mixture (20 µl) contained 1 µl of RT product (cDNA transcribed from 50 ng of total RNA), 10 µl of TaqMan® Universal PCR Master Mix (Applied Biosystems, Austin, TX, USA) and 1 µl of the appropriate TaqMan® Gene Expression Assay (Applied Biosystems, Austin, TX, USA) containing primers and probe for the mRNA of interest. The mixture was initially incubated at 95 °C for 10 min, followed by 40 cycles of 95 °C for 15 s and 60 °C for 60 s. PCR reactions were carried out on a 7500 Real-Time PCR System (Applied Biosystems, Austin, TX, USA) in triplicate. Samples were normalized relative to human large ribosomal protein (RPLPO) which served as endogenous control. mRNA levels were compared as 2^{-ΔCt} values or relative gene expression levels were calculated using the 2^{-ΔΔCt} method (Schmittgen and Livak, Nat Protoc. 3:1101-1108, (2008)).

For relative quantification of miRNAs, total RNA was reverse transcribed using the TaqMan MicroRNA Reverse Transcription Kit (Applied Biosystems, Austin, TX, USA) according to the manufacturer's protocol. 10 ng of total RNA in a total volume of 15 µl were transcribed to cDNA using specific primers for each respective miRNA. The reverse transcription (RT) reaction was performed by sequential incubation at 16°C for 30 min, 42°C for 30 min, and 85°C for 5 min. Each quantitative real-time PCR reaction mixture (20 µl) contained 1.33 µl of RT product, 10 µl of TaqMan® Universal PCR Master Mix No AmpErase® UNG (Applied Biosystems, Austin, TX, USA) and 1 µl of the appropriate TaqMan® MicroRNA Assay (Applied Biosystems, Austin, TX, USA) containing primers and probe for the miRNA of interest. TaqMan® MicroRNA assays were used as follows. The mixture was initially incubated at 95 C for 10 min, followed by 40 cycles of 95 C for 15 s and 60 C for 60 s. PCR reactions were carried out on a 7500 Real-Time PCR System (Applied Biosystems, Austin, TX, USA) in triplicate. Samples were normalized relative to human small nucleolar RNA RNU38b, which served as endogenous control. mRNA levels were compared as 2^{-ΔCt} values or relative gene expression levels were calculated using the 2^{-ΔΔCt} method (Schmittgen and Livak, Nat Protoc. 3:1101-1108, (2008)).

TaqMan probes used were: SIRT4: Part No.: 4331182, Assay-ID: Hs00202033_m1*; RPLPO: Part No.: 4331182 Assay-ID: Hs99999902_m1*; hsa-mir-15b: Part No.: 4427975, Assay-ID: 000390; RNU38B: Part No.: 4427975, Assay-ID: 001004; RNU24: Part No.: 4427975, Assay-ID: 001001. Results are given as means ± s.d. P-values below 0.05 were considered significant. To evaluate statistical significance, Student's t-tests or Oneway ANOVA rank tests were performed.

Isolation of CD34⁺ progenitor cells and expression analysis of SIRT4 and miR-15b. Mononucleated cells were collected from unbilical cord blood, from bone marrow of healthy adult donors, or from peripheral blood of healthy adult donors following mobilization with G-CSF. Informed consent was obtained from all donors or the donors' mothers respectively, and the investigation has been approved by the University of Düsseldorf Ethics Committee. CD34⁺ hematopoietic progenitor cells (HPC) were enriched with immunomagnetic beads specific for CD34 (Miltenyi Biotech, Bergisch-Gladbach) to a purity of > 90%. All other fractions of hematopoietic stem and progenitor cells, as well as NK cells, were sorted to a purity > 99%.

Total RNA was isolated with the *mirVana* Kit (Ambion) and the fraction of small RNAs was collected separately, cDNA was generated using MMLV Reverse Transkriptase and oligo(dT) primers (Promega) according to the manufacturer's instructions. Expression of genes was measured with TaqMan Gene Expression Assays and normalized to *GAPDH* as a housekeeping gene. Mean values were calculated after normalization and significance was determined with two-tailed t-tests. For miRNA analysis, 5,000 cells were lysed at 95°C for 5 min and used directly for reverse transcription. Expression of micro RNA miR-15b was assessed via the TaqMan mircoRNA Megaplex Assay (Applied Biosystems) and normalized to the mean of all expressed miRNAs.

Microarray Analysis. Microarray analysis were done using the "Quick Amp Labeling Kit, one color" (Agilent Technologies Cat.-No 5190-0442) and the RNA Spike In Kit, one color (Agilent Technologies Cat.-No 5188-5282)following the manufacturer's protocol. We used 500 ng of total RNA as starting material for cDNA synthesis. The cDNA synthesis and in vitro transcription (IVT) were performed according the manufacturer's instructions. The quantity and labeling efficiency of the labeled cRNA were measured photometrical (NanoDrop ND-1000, Fisher Scientific). For gene expression analysis the labeled cRNA was hybridized onto "Whole Human Genome 4x44K Microarrays" (Agilent Technologies, Cat.-No G4112F) for 17 hours at 65°C and 10 rpm in the hybridization oven (Agilent Technologies). Washing of the arrays were performed in according to the manufacturer's recommendation. The Cy3 fluorescence intensities were detected by scanning the arrays using the Agilent DNA microarray scanner (G2505B). The resulting image files were analyzed with Feature extraction software. Raw data files were processed and analysed using the GeneSpring Software Version 11 (Agilent).

Example 1: Analysis of SIRT4 expression in human skin samples. Skin samples were from healthy human volunteers after approval had been obtained from the Ethics Committee of the Heinrich-Heine-University. The study has been conducted according to the ethical rules stated in the Declaration of Helsinki Principles and the ICH GCP guideline was observed insofar as applicable. Healthy human volunteers (15 to 16 for each age group) were enrolled after written informed consent. The age range of the first group was 18 to 25 years and of the second group 60-66 years. All individuals were non-smokers and had no history of any severe skin disease, especially no photosensitivity disorders. Skin types ranged from Fitzpatrick type I to IV. In each volunteer, one 4 mm punch biopsy was taken from neck (i.e. a skin side chronically exposed to UV radiation) and from buttock skin (i.e. a skin side chronically protected from UV radiation), as previously described (Berneburg et al., Photochem Photobiol. 66:271-275, (1997)). RNA from human biopsies was isolated as described (Grether-Beck et al., Exp Dermatol. 17:771-779, (2008)). For assessment of gene expression total RNA was extracted from frozen biopsies and gene expression measured by semiquantitative RT-PCR as previously described. In brief, for isolation of RNA from frozen skin biopsies, 600 µl lysis buffer from PeqGold Total RNA Kit (PeqLab, Erlangen, Germany) were added and the samples were disrupted in a MixerMill MM300 (Retsch, Haan, Germany) three times for 3 min with 30 Hz. 50 ng total RNA were used for cDNA synthesis. Alternatively, skin tissue was disrupted and homogenized in 600 µl RLT+ buffer (+ 1% β-mercaptoethanol) for 60-90 seconds using an ultra-turrax.

PCR reactions were performed in an Opticon 1 (MJ Research, Waltham, MA, USA) using Sybr QPCR Supermix w. Rox (Invitrogen, Karlsruhe, Germany). PCR conditions were as follows: activation of hot start taq polymerase 94° C 15 minutes; denaturation 95° C, 20 seconds; annealing, 55° C, 20 seconds; extension, 72°C for 30 seconds. Each sample was subjected to PCR in double using the appropriate primer pairs for 35 cycles. For comparison of relative expression in real time PCR of unexposed and exposed skin the 2^{(-ΔΔC(T))} method was used (Livak and Schmittgen, Methods. 25:402-408, (2001)). Primer for SIRT4 (Haigis et al., Cell. 126:941-954, (2006)) were derived from the sequence NM_012240.2 (5'-GCACACTGGGCTTTGAGC-3') (SEQ ID NO:16) and 5'-CACAATCCAAGCACAGGA-3' (SEQ ID NO:17)). Samples were normalized relative to 18S rRNA which served as endogenous control (primer used: 5'-GCCGCTAGAGGTGAAATTCTTG-3' (SEQ ID NO:18) and 5'-CATTCTTGGCAAATGCTTTCG-3' (SEQ ID NO:19); McCallum and Maden, Biochem J. 232:725-733, (1985)). Alternatively, analysis of SIRT4 mRNA levels in human skin samples was also performed essentially as described above for the analysis of cultured and irradiated cells. Results are given as means ± s.e.m. Wilcoxon signed rank test was employed for statistical analysis, and p values of less than 0.05 were considered statistically significant.

Example 2: Inhibition of basal and senescence-associated SIRT4 expression by transfection of miR-15b specific "mimics". Control miRNA mimics or oligonucleotides mimicking endogenous miR-15b function of SEQ ID NOs:14 and 15, ("miR-15b mimics" MSY0000417 and MSY0004586, respectively, Qiagen, Hilden) were transfected into MCF7 cells using the HiPerFect transfection reagent (Qiagen) essentially as described by the provider. Thereafter cellular TACC3 depletion and hence premature senescence was triggered by the addition of doxycyclin as described above. SIRT4 mRNA levels were determined four days later by qPCR as described above. Results are given as means ± s.d. P-values below 0.05 were considered significant. To evaluate statistical significance, Student's t-tests were performed.

### Sequence Listing (Free Text)

| SEQ ID NO: | Description |
|---|---|
| 1-13 | 3'-UTR of the *SIRT4* gene in vertebrates |
| 14 and 15 | miR-15b miRNA mimic |
| 16-19 | primer |

## Claims

1. A compound for upregulating cellular miR-15b expression, a pharmaceutical composition and medicament comprising said compound for use in preventing or treating cellular senescence and tissue aging.

2. The compound, pharmaceutical composition and medicament for use in preventing or treating cellular senescence and tissue aging of claim 1, wherein the compound is a miR-15b miRNA mimic.

3. The compound, pharmaceutical composition and medicament for use in preventing or treating cellular senescence and tissue aging of claim 2, wherein the miR-15b miRNA mimic binds to the miR-15b binding site UGCUGCUA or the complement thereof within the 3'-UTR of the *SIRT4* gene.

4. The compound, pharmaceutical composition and medicament for use in preventing or treating cellular senescence and tissue aging of claim 2 or 3, wherein the miR-15b miRNA mimic comprises a nucleotide sequence having the sequence of SEQ ID NO:14 or 15, or the complement thereof.

5. The compound, pharmaceutical composition and medicament for use in preventing or treating cellular senescence and tissue aging of any one of claims 2 to 4, wherein the miR-15b miRNA mimic is comprised in a nucleic acid construct, vector or viral vector.

6. The compound, pharmaceutical composition and medicament for use in preventing or treating cellular senescence and tissue aging of any one of claims 1 to 5, which is for preventing or treating skin aging.

7. Use of the compound for upregulating cellular miR-15b expression as defined in any one of claims 1 to 5 for preparing a medicament for preventing or treating cellular senescence and tissue aging.

8. The use of claim 7, wherein the medicament is for preventing or treating skin aging

9. An *in-vitro* method for preventing or treating cellular senescence and tissue aging, which comprises contacting the cells or tissue with the compound for upregulating cellular miR-15b expression as defined in any one of claims 1 to 5.

10. A pharmaceutical or cosmetical method for preventing or treating cellular senescence and tissue aging in a subject which comprises administering to the subject an effective amount of the compound for upregulating cellular miR-15b expression as defined in any one of claims 1 to 5.

11. The pharmaceutical or cosmetical method of claim 10, which is for preventing or treating skin aging.

12. A cosmetical composition for preventing or treating cellular senescence and tissue aging comprising the compound for upregulating cellular miR-15b expression as defined in any one of claims 1 to 5.

13. The cosmetical composition of claim 12, which is for preventing or treating skin aging.


SEQUENCE LISTING

<110>  IUF Leibnitz-Institut fuer umweltmedizinische Forschung
       gGmbH

<120>  Upregulation of miR-15b for Preventing Cellular Senescence and
       Tissue Aging

<130>  111867ep

<160>  19

<170>  PatentIn version 3.3

<210>  1
<211>  107
<212>  RNA
<213>  Homo sapiens

<400>  1
ccacagccug auauuccaga accuggaaca gggacuuuca cuugaaucuu gcugcuaaau      60

guaaaugccu ucucaaauga cagauuccag uucccauuca acagagu                   107


<210>  2
<211>  107
<212>  RNA
<213>  Pan troglodytes

<400>  2
ccacagccug auauuccaga accuggaaca gggacuuuca cuugaaucuu gcugcuaaau      60

guaaaugccu ucucaaauga cagauuccag uucccauuca acagagu                   107


<210>  3
<211>  93
<212>  RNA
<213>  Mus musculus

<400>  3
cagcacucug auguccaaag gcuggaaaug aacuuuccuc ugaguuccgc ugcucaaggu      60

aaagagaaag cccucucagg uaaagggguc uag                                   93


<210>  4
<211>  97
<212>  RNA
<213>  Rattus norvegicus

<400>  4
cagcacccug auguccaaag gcuggaaagg acuuuccuuu gggucccgcu gcuaaaggua      60

aagagagccc uuucagauuc cugguacacc uaguagu                               97


<210>  5
<211>  74
<212>  RNA
<213>  Cavia porcellus

<400>  5
ccacagccug cccauccuag gccagaacag ggaccuuacu uccaauccug cuguugaagg      60

uaaaugcccu uuua                                                          74

<210>  6
<211>  101
<212>  RNA
<213>  Oryctolagus cuniculus

<400>  6
cagccugaug uuccugaucc agaaacuggg ucuuucaacu ugaaucugcu gccaaaggua      60

ggugccuucu cagguccuag uaccacguga cuaagaguag g                         101

<210>  7
<211>  32
<212>  RNA
<213>  Sorex araneus

<400>  7
ccgcggccac uugcuccugc gcccccgacg gg                                    32

<210>  8
<211>  103
<212>  RNA
<213>  Canis familiaris

<400>  8
ccacaaccug auguuucuga gccagaaacu gggacuuuca cuuuaauccu auuaaaggua      60

aguuaaaggc uuucuuagau accaguuucc aguuugcauu caa                       103

<210>  9
<211>  101
<212>  RNA
<213>  Equus caballus

<400>  9
ccacagccug auguuccugc gccggaaacu ggaauuucca cuugaauccu gcugcuaaag      60

guaaaugccu ucucagauau cagaugccag uuuccaguca a                         101

<210>  10
<211>  88
<212>  RNA
<213>  Bos taurus

<400>  10
ccacagccug auguucugga gccggaaacu ggggcuuuca cuuuaauucu gcugcuaagg      60

guaaaaaacc uuagauacca gauuccag                                         88

<210>  11
<211>  109
<212>  RNA
<213>  Dasypus novemcinctus

<400>  11
cuacagagac auguucccaa gccagaaaca gggauuuuca cuggaauucu gcugcuaaag      60

auaaaugccu ucacagacau cagauuccag cucccacuca acagagggc 109

<210> 12
<211> 32
<212> RNA
<213> Ornithorhynchus anatinus

<400> 12
ccggagccuc acgggccccg cucggacagg ga 32

<210> 13
<211> 15
<212> RNA
<213> Anolis carolinensis

<400> 13
cccagcucgg caccc 15

<210> 14
<211> 22
<212> RNA
<213> Artificial

<220>
<223> miRNA Mimic

<400> 14
uagcagcaca ucaugguuua ca 22

<210> 15
<211> 22
<212> RNA
<213> Artificial

<220>
<223> miRNA mimic

<400> 15
cgaaucauua uuugcugcuc ua 22

<210> 16
<211> 18
<212> DNA
<213> Artificial

<220>
<223> Primer

<400> 16
gcacactggg ctttgagc 18

<210> 17
<211> 18
<212> DNA
<213> Artificial

<220>
<223> primer

<400>  17
cacaatccaa gcacagga                                                    18


<210>  18
<211>  22
<212>  DNA
<213>  Artificial

<220>
<223>  primer

<400>  18
gccgctagag gtgaaattct tg                                               22


<210>  19
<211>  21
<212>  DNA
<213>  Artificial

<220>
<223>  primer

<400>  19
cattcttggc aaatgctttc g                                                21